# EUROPÄISCHE PATENTANMELDUNG

(11) **EP 1 982 717 A1**
(43) Veröffentlichungstag der Anmeldung: **22.10.2008**
(21) Anmeldenummer: 07106647.6
(22) Anmeldetag: 20.04.2007
(51) Int. Cl.: A61K 31/4196, A61K 31/44, A61P 31/10

(54) **Verwendung von Fungiziden zur Behandlung von Fischmykosen**

(71) Anmelder: Bayer CropScience AG, 40789 Monheim (DE)
(72) Erfinder: Häuser-Hahn, Dr. Isolde, 51375 Leverkusen (DE); Stenzel, Dr. Klaus, 40595 Düsseldorf (DE); Wachendorff-Neumann, Dr. Ulrike, 56566 Neuwied (DE)

(57) **Zusammenfassung**

Verfahren zum Schutz von Fischen und Wirbellosen und all deren Entwicklungsstadien vor Mykosen, hervorgerufen durch Pilze der Gattungen *Saprolegnia, Aphanomyces, Achlyaflagellata* und anderer in Aquakulturen bedeutsamer Arten durch den Einsatz von ausgewählten Fungiziden, die als agrochemische Wirkstoffe bereits bekannt sind. Dieser Einsatz führt zu einer Hemmung bzw. Abtötung pathogener Pilze.
Das Mittel, enthaltend mindestens ein Fungizid ausgewählt aus oben genannter Gruppe zur Anwendung in der Fischzucht- und -haltung eignet sich zur Prophylaxe und Therapie von Erkrankungen von Fischen in der Aquakultur, in Zuchtteichen, Zuchttanks, Aquarien, natürlichen Sportfischgewässern, Teichen, und marinen Fischzuchten. Die zugehörigen Anwendungsformen sind der Zusatz zum Wasser und Futter und Direktapplikation. Der Zusatz des erfindungsgemäßen Mittels zum Wasser vermindert Laich- und Fischverpilzungen.

## Beschreibung

Die Erfindung betrifft ein Verfahren zur Prophylaxe und Therapie von Mykosen bei Fischen und Wirbellosen und all deren Entwicklungsstadien, hervorgerufen durch Pilze der Gattungen *Saprolegnia, Achlya, Aphanomyces* und anderer in Aquakulturen bedeutsamer Arten (nachfolgend pathogene Pilze genannt) durch den Einsatz von mindestens einem Fungizid, ausgewählt aus folgender Gruppe 1) Inhibitoren der Nukleinsäuresynthese, 2) Inhibitoren von Mitose und Zellteilung, 3.1) Inhibitoren am Komplex I der Atmungskette, 3.2) Inhibitoren am Komplex II der Atmungskette, 4) Entkoppler, 5) Inhibitoren der ATP Produktion, 6) Inhibitoren der Aminosäure- und Protein-Biosynthese, 7) Inhibitoren der Signaltransduktion, 8) Inhibitoren der Lipid- und Membran-Synthese, 9) Inhibitoren der Ergosterol-Biosynthese, 10) Inhibitoren der Zellwandsynthese, 11) Inhibitoren der Melanin-Biosynthese, 12) Resistenzinduktoren, 13) Verbindungen mit Multisite-Aktivität, 14) eine Verbindung aus der folgenden Liste: 2,3,5,6-Tetrachlor-4-(methylsulfonyl)pyridine, 2,3-Dibutyl-6-chloro-thieno[2,3-d]pyrimidin-4(3H)one, 2-Butoxy-6-iod-3-propyl-benzopyran-4-on, 2-chloro-N-(2,3-dihydro-1,1,3-trimethyl-1H-inden-4-yl)-3-pyridincarboxamide, 3,4,5-Trichlorpyridine-2,6-dicarbonitrile, 3-[5-(4-Chlorphenyl)-2,3-dimethylisoxazolidin-3-yl]pyridine, 8-hydroxyquinoline-sulfate, Benthiazole, Bethoxazin, Capsimycin, Carvone, Chinomethionat, Cufraneb, Cyflufenamid, Cymoxanil, Cyprosulfamide, Dazomet, Debacarb, Dichlorophen, Diclomezine, Dicloran, Difenzoquat, Difenzoquat methylsulphate, Diphenylamine, Ferimzone, Flumetover, Fluopicolide, Fluoroimide, Flusulfamide, Fosetyl-Al, Fosetyl-calcium, Fosetyl-natrium, Hexachlorobenzene, Irumamycin, Isotianil, Methasulfocarb, Methyl isothiocyanate, Metrafenone, Mildiomycin, N-(4-Chlor-2-nitrophenyl)-N-ethyl-4-methylbenzenesulfonamide, N-(4-Chlorbenzyl)-3-[3-methoxy-4-(prop-2-yn-1-yloxy) phenyl]propanamide, N-(6-methoxy-3-pyridinyl)-cyclopropanecarboxamide, N-[(4-Chlorphenyl) (cyano)methyl]-3-[3-methoxy-4-(prop-2-yn-1-yloxy)-phenyl]-propanamide, N-[(5-Brom-3-chlor-pyridin-2-yl)methyl]-2,4-dichlomicotinamide, N-[1-(5-Brom-3-chlorpyridin-2-yl)ethyl]-2,4-dichlomicotinamide, N-[1-(5-Brom-3-chlorpyridin-2-yl)ethyl]-2-fluoro-4-iodonicotinamide, Natamycin, Nickel-dimethyldithiocarbamate, Nitrothal-isopropyl, Octhilinone, Oxyfenthiin, Pentachlorophenol and salts, Phosphorous acid and its salts, Propamocarb-Fosetyl, Propanosine-sodium, Proquinazid, Pyrrolnitrine, Quintozene, S-allyl-5-amino-2-isopropyl-4-(2-methylphenyl)-3-oxo-2,3-dihydro-1H-pyrazole-1-carbothioate, Tecloftalam, Tecnazene, Triazoxide, Trichlamide, Zarilamid.

Die Erfindung betrifft ebenfalls ein Mittel, enthaltend mindestens ein Fungizid ausgewählt aus folgender Gruppe 1) Inhibitoren der Nukleinsäuresynthese, 2) Inhibitoren von Mitose und Zellteilung, 3.1) Inhibitoren am Komplex I der Atmungskette, 3.2) Inhibitoren am Komplex II der Atmungskette, 4) Entkoppler, 5) Inhibitoren der ATP Produktion, 6) Inhibitoren der Aminosäure- und Protein-Biosynthese, 7) Inhibitoren der Signaltransduktion, 8) Inhibitoren der Lipid- und Membran-Synthese, 9) Inhibitoren der Ergosterol-Biosynthese, 10) Inhibitoren der Zellwandsynthese, 11) Inhibitoren der Melanin-Biosynthese, 12) Resistenzinduktoren, 13) Verbindungen mit Multisite-Aktivität, 14) eine Verbindung aus der folgenden Liste: 2,3,5,6-Tetrachlor-4-(methylsulfonyl)pyridine, 2,3-Dibutyl-6-chloro-thieno[2,3-d]pyrimidin-4(3H)one, 2-Butoxy-6-iod-3-propyl-benzopyran-4-on, 2-chloro-N-(2,3-dihydro-1,1,3-trimethyl-1H-inden-4-yl)-3-pyridincarboxamide, 3,4,5-Trichlorpyridine-2,6-dicarbonitrile, 3-[5-(4-Chlorphenyl)-2,3-dimethylisoxazolidin-3-yl]pyridine, 8-hydroxyquinoline-sulfate, Benthiazole, Bethoxazin, Capsimycin, Carvone, Chinomethionat, Cufraneb, Cyflufenamid, Cymoxanil, Cyprosulfamide, Dazomet, Debacarb, Dichlorophen, Diclomezine, Dicloran, Difenzoquat, Difenzoquat methylsulphate, Diphenylamine, Ferimzone, Flumetover, Fluopicolide, Fluoroimide, Flusulfamide, Fosetyl-Al, Fosetyl-calcium, Fosetyl-natrium, Hexachlorobenzene, Irumamycin, Isotianil, Methasulfocarb, Methyl isothiocyanate, Metrafenone, Mildiomycin, N-(4-Chlor-2-nitrophenyl)-N-ethyl-4-methylbenzenesulfonamide, N-(4-Chlorbenzyl)-3-[3-methoxy-4-(prop-2-yn-1-yloxy) phenyl]propanamide, N-(6-methoxy-3-pyridinyl)-cyclopropanecarboxamide, N-[(4-Chlorphenyl) (cyano)methyl]-3-[3-methoxy-4-(prop-2-yn-1-yloxy)-phenyl]-propanamide, N-[(5-Brom-3-chlorpyridin-2-yl)methyl]-2,4-dichlomicotinamide, N-[1-(5-Brom-3-chlorpyridin-2-yl)ethyl]-2,4-dichlomicotinamide, N-[1-(5-Brom-3-chlorpyridin-2-yl)ethyl]-2-fluoro-4-iodonicotinamide, Natamycin, Nickel-dimethyldithiocarbamate, Nitrothal-isopropyl, Octhilinone, Oxyfenthiin, Pentachlorophenol and salts, Phosphorous acid and its salts, Propamocarb-Fosetyl, Propanosine-sodium, Proquinazid, Pyrrolnitrine, Quintozene, S-allyl-5-amino-2-isopropyl-4-(2-methylphenyl)-3-oxo-2,3-dihydro-1H-pyrazole-1-carbothioate, Tecloftalam, Tecnazene, Triazoxide, Trichlamide, Zarilamid zur Prophylaxe und Therapie von Mykosen bei Fischen und Wirbellosen und all deren Entwicklungsstadien, hervorgerufen durch Pilze der Gattungen *Saprolegnia, Achlya, Aphanomyces* und anderer in Aquakulturen bedeutsamer Arten (nachfolgend pathogene Pilze genannt). Es findet Anwendung in der Fischzucht und in der Fischhaltung, speziell für den prophylaktischen und therapeutischen Einsatz bei erkrankten und gestressten Fischen.

Die Erfindung betrifft ferner den Einsatz eines Mittels zur antimykotischen Behandlung von Fischlaich.

Aufgrund des weltweit steigenden Bedarfs an Fisch wird dieser zunehmend in Aquakultur gehalten. Beispielhaft, aber nicht begrenzend, seien folgende relevante Fischspezies für die Aquakultur genannt: Wels, Forelle, Lachs, Pangasius und Barsche. Aufgrund der Nähe der Fische zueinander sind solche Aquakulturen extrem anfällig gegenüber Pathogenen, insbesondere pathogenen Pilzen. Pathogene Pilze, die sowohl Fische als auch Fischeier befallen, gehören zu folgenden Gattungen: *Saprolegnia hypogyna, S. ferax, S. australis, S. declina, S. longicaulis, S. mixta, S. parasitica, S. sporangium, S. variabilis, Aphanomyces invadans* und *Achlyaflagellata spp.*

Durch Mykosen entstehen bei der Züchtung und Haltung von Nutz- und Zierfischen sowie von Krebsen und anderen Wirbellosen hohe wirtschaftliche Verluste *(*Bruno,D. W., Wood, B.P., 1999: Saprolegnia and other Oomycetes*. In:* Woo, P.T.K., Bruno, D.W. (Hrsg): Fish Diseases and Disorder. Vol.3 Viral, Bacterial and fungal infections. CAB International, Wallingford*).*

Bisher sind jedoch nur wenige Substanzen bekannt, die zur Bekämpfung von Fischmykosen geeignet sind.

In der Vergangenheit wurde oftmals Malachitgrün als Wirkstoff zur Prophylaxe und Therapie von Mykosen verwendet. Aufgrund seiner kanzerogenen, mutagenen und teratogenen Eigenschaften wird diese Substanz in Deutschland jedoch nur zur Behandlung von Fischeiern geduldet, ist aber nicht zur Therapie von Fischen zugelassen *(*Meyer,F.P.; Jorgenson.TA., 1983: Teratological and other effects of malachite green on development of rainbow trout and rabbits. Trans.Am.Fish.Soc. 112, 818-824, *(Bundesinstitut fur gesundheitlichen Verbraucherschutz und Veterinärmedizin, 2002).* Da Malachitgrün ein Farbstoff ist, kann es zu Verfärbungen des Wassers und der behandelten Fische kommen. Desweiteren besitzt Malachitgrün eine lange Halbwertszeit, so dass es im später zu verzehrenden Fisch zu Rückständen kommen kann (D.J. Alderman in Journal of Fish Diseases 8. (1985) 289-298).

Heute wird weiterhin Formalin eingesetzt, das zwar gewisse fungizide Wirkung gegen pathogene Pilze hat, jedoch in der praktischen Anwendung nicht zufrieden stellend ist und darüber hinaus Probleme bei der Arbeitssicherheit bereitet, insbesondere in geschlossenen Systemen.

In DE 10237740 wird die Verwendung von natürlichen und synthetischen Huminstoffen in der Fischzucht beschrieben.

Aus DE 2 037 610 ist bereits bekannt, dass gewisse Benzylimidazole in vitro gegen *Saprolegnia parasitica* wirken. Es wird aber nichts über eine potentielle Anwendung in der Fischzucht erwähnt.

In CN 1472448 werden fungizid wirksame Kräuterformulierungen zur Verwendung als Mittel gegen Saprolegniasis bei Fischen, Shrimps und Krabben beschrieben. Die Formulierungen enthalten Galla Chinensis 40-70, Cortex Phellodendri (Phellodendron chinense and/or Phellodendron amurense) 10-30, Paeonia suffruticosa bark 10-30, and Houttuynia cordata 10-30 wt%.

Auch Teeextrakte, enthaltend diverse Polyphenole können eingesetzt werden (JP 3698745).

In der WO 2004002574 werden Enzymmischungen, enthaltend Glucanasen zur Prophylaxe und Therapie von Mykosen in Fischen und Entwicklungsstadien davon (Eiern) beschrieben.

In der WO 98 05311 wird Bromopol (Brom-2-Nitropropan-1,3-diol) zur Bekämpfung verschiedener Krankheiten aquatischer Organismen, insbesondere Salmonen und ihrer Eier verwendet.

Des weiteren ist die Verwendung von Chlordioxid (WO95 18534), 3-Phenoxycarbonylmethoxy-1,1,2-trüod-1-propen (JP 57116012) und 2-Pyridinethiol 1-oxide (JP 47019191) zur Verwendung gegen Saprolegnia-Erkrankungen bekannt.

Aus der WO97/006690 ist die Verwendung von Kresoximmethyl zur Bekämpfung von Fischmykosen bekannt.

Allerdings ist die Wirksamkeit dieser Stoffe gerade in geringeren Dosen oft nicht ausreichend. Zudem ist eine genaue Dosierung, insbesondere von Kräuter- oder Teeextrakten oder Enzymmischungen aufgrund deren schwankenden Wirkstoffgehalten schwierig. Des weiteren ist die Anwendung dieser Präparate nur für einzelne Fischarten und -krankheiten geeignet. Zudem ist die Anwendung der Präparate oftmals auf einen Zeitraum lange vor der Befischung (,Ernte') aufgrund ihrer toxischen Eigenschaften beschränkt, da lange Wartezeiten zwischen der Anwendung und dem Verzehr der Fische erforderlich sind.

Zudem haben diese Stoffe toxische Nebenwirkungen. Die Verwendung von z.B. Formalin, Chloramin T und Malachitgrün-oxalat ist oft mit einer Reihe von Nebenwirkungen und Risiken verbunden, so z.B. weist es karzinogenes, mutagenes, chromosomenschädigendes, teratogenes Potential auf, wirkt als Atemgift, des weiteren treten histopathologische Folgen und Multiorganschäden sowie signifikante Alterationen biochemischer Blutparameter auf (Sanchez et. Al 1998; Srivastava et al. 2004; aus Dissertation St. Heidrich, 2005).

Neben den reinen Desinfektionsmitteln sind auch andere Substanzen in Gebrauch. Dazu gehören Essigsäure (für Tauchbehandlungen), Natrium- und Kalziumchlorid (als osmoregulatorische Behandlungen), Natriumkarbonat und Kohlendioxid (zur Betäubung von Fischen) sowie Natriumsulfit (zur Verbesserung des Eischlupfes) und Povidone iodone (zur Desinfektion der Fischlaichoberfläche).

Die Ursachen für die Nachteile von herkömmlichen Prophylaktika und Therapeutika resultieren aus deren langjährigem ausgedehnten und teilweise unbedachten Einsatz, z. B. von Antibiotika, Chemotherapeutika sowie Triphenylfarbstoffen u. a. Wirkstoffen zur Beseitigung von bakteriellen, parasitären und umweltbedingten Erkrankungen sowie zur Ertragssteigerung. Aus diesem Einsatz entwickelten sich z. T. ungünstige Resistenzsituationen für die Behandlung von Erkrankungen bei Tier und Mensch. Desweiteren ist der Einsatz herkömmlicher Mittel oft mit starken Nebenwirkungen, Risiken und Umweltschädigungen verbunden.

Demzufolge besteht auf dem Feld der Aquakultur ein erhöhter Bedarf an effizienten Mitteln, die Pathogene kontrollieren, die die Produktivität kommerzieller Fischherstellung herabsetzen. Beim Auftreten von Fischkrankheiten spielt ein frühzeitiges Eingreifen mit vorwiegend prophylaktischem Charakter sowie ein Vermeiden und Mildem von krankheitsauslösenden Faktoren eine immer größere Rolle. Aus diesem Grund kommt der Suche nach alternativen Behandlungsmöglichkeiten eine hervorragende Bedeutung zu. Die einsetzbaren Mittel sollten ein breites Erregerspektrum kontrollieren und bestehende Sicherheitsrichtlinien erfüllen. Die vorliegende Erfindung erfüllt diese Kriterien und liefert weitere Vorteile.

Die im erfindungsgemässen Verfahren verwendeten Fungizide sind als agrochemische Wirkstoffe bereits bekannt (vgl. z.B. Pesticide Manual, 13th edition).

Es wurde nun überraschenderweise gefunden, dass mindestens ein Fungizid, ausgewählt aus folgender Gruppe 1) Inhibitoren der Nukleinsäuresynthese, 2) Inhibitoren von Mitose und Zellteilung, 3.1) Inhibitoren am Komplex I der Atmungskette, 3.2) Inhibitoren am Komplex II der Atmungskette, 4) Entkoppler, 5) Inhibitoren der ATP Produktion, 6) Inhibitoren der Aminosäure- und Protein-Biosynthese, 7) Inhibitoren der Signaltransduktion, 8) Inhibitoren der Lipid- und Membran-Synthese, 9) Inhibitoren der Ergosterol-Biosynthese, 10) Inhibitoren der Zellwandsynthese, 11) Inhibitoren der Melanin-Biosynthese, 12) Resistenzinduktoren, 13) Verbindungen mit Multisite-Aktivität, 14) eine Verbindung aus der folgenden Liste: 2,3,5,6-Tetrachlor-4-(methylsulfonyl)pyridine, 2,3-Dibutyl-6-chloro-thieno[2,3-d]pyrimidin-4(3H)one, 2-Butoxy-6-iod-3-propyl-benzopyran-4-on, 2-chloro-N-(2,3-dihydro-1,1,3-trimethyl-1H-inden-4-yl)-3-pyridincarboxamide, 3,4,5-Trichlorpyridine-2,6-dicarbonitrile, 3-[5-(4-Chlorphenyl)-2,3-dimethylisoxazolidin-3-yl]pyridine, 8-hydroxyquinoline-sulfate, Benthiazole, Bethoxazin, Capsimycin, Carvone, Chinomethionat, Cufraneb, Cyflufenamid, Cymoxanil, Cyprosulfamide, Dazomet, Debacarb, Dichlorophen, Diclomezine, Dicloran, Difenzoquat, Difenzoquat methylsulphate, Diphenylamine, Ferimzone, Flumetover, Fluopicolide, Fluoroimide, Flusulfamide, Fosetyl-Al, Fosetyl-calcium, Fosetyl-natrium, Hexachlorobenzene, Irumamycin, Isotianil, Methasulfocarb, Methyl isothiocyanate, Metrafenone, Mildiomycin, N-(4-Chlor-2-nitrophenyl)-N-ethyl-4-methylbenzenesulfonamide, N-(4-Chlorbenzyl)-3-[3-methoxy-4-(prop-2-yn-1-yloxy) phenyl]propanamide, N-(6-methoxy-3-pyridinyl)-cyclopropanecarboxamide, N-[(4-Chlorphenyl)(cyano)methyl]-3-[3-methoxy-4-(prop-2-yn-1-yloxy)-phenyl]-propanamide, N-[(5-Brom-3-chlorpyridin-2-yl)methyl]-2,4-dichlomicotinamide, N-[1-(5-Brom-3-chlorpyridin-2-yl)ethyl]-2,4-dichlomicotinamide, N-[1-(5-Brom-3-chlorpyridin-2-yl)ethyl]-2-fluoro-4-iodonicotinamide, Natamycin, Nickel-dimethyldithiocarbamate, Nitrothal-isopropyl, Octhilinone, Oxyfenthiin, Pentachlorophenol and salts, Phosphorous acid and its salts, Propamocarb-Fosetyl, Propanosine-sodium, Proquinazid, Pyrrolnitrine, Quintozene, S-allyl-5-amino-2-isopropyl-4-(2-methylphenyl)-3-oxo-2,3-dihydro-1H-pyrazole-1-carbothioate, Tecloftalam, Tecnazene, Triazoxide, Trichlamide, Zarilamid sich sehr gut dazu eignet, Mykosen bei Fischen und Wirbellosen und all deren Entwicklungsstadien, hervorgerufen durch Pilze der Gattungen *Saprolegnia, Achlya, Aphanomyces* sowohl vorzubeugen als auch zu behandeln, insbesondere Pilzerkrankungen, hervorgerufen durch *Saprolegnia spp..*

Die Verwendung der genannten Fungizide bei der Behandlung weist gegenüber den aus dem Stand der Technik bekannten Stoffen bzw. Behandlungsmethoden folgende Vorteile auf: sie zeigen eine gute Wirksamkeit und reichem sich nicht in unerwünschtem Maße im Fischorganismus an, sie haben günstige öko- und sonstige toxikologische Eigenschaften und zeigen keine unakzeptablen Auswirkungen auf die Lebensgemeinschaften.

Die Erfindung betrifft somit die Verwendung von mindetsens einem Fungizid ausgewählt aus folgender Gruppe 1) Inhibitoren der Nukleinsäuresynthese, 2) Inhibitoren von Mitose und Zellteilung, 3.1) Inhibitoren am Komplex I der Atmungskette, 3.2) Inhibitoren am Komplex II der Atmungskette, 4) Entkoppler, 5) Inhibitoren der ATP Produktion, 6) Inhibitoren der Aminosäure- und Protein-Biosynthese, 7) Inhibitoren der Signaltransduktion, 8) Inhibitoren der Lipid- und Membran-Synthese, 9) Inhibitoren der Ergosterol-Biosynthese, 10) Inhibitoren der Zellwandsynthese, 11) Inhibitoren der Melanin-Biosynthese, 12) Resistenzinduktoren, 13) Verbindungen mit Multisite-Aktivität, 14) eine Verbindung aus der folgenden Liste: 2,3,5,6-Tetrachlor-4-(methylsulfonyl)pyridine, 2,3-Dibutyl-6-chloro-thieno[2,3-d]pyrimidin-4(3H)one, 2-Butoxy-6-iod-3-propyl-benzopyran-4-on, 2-chloro-N-(2,3-dihydro-1,1,3-trimethyl-1H-inden-4-yl)-3-pyridincarboxamide, 3,4,5-Trichlorpyridine-2,6-dicarbonitrile, 3-[5-(4-Chlorphenyl)-2,3-dimethylisoxazolidin-3-yl]pyridine, 8-hydroxyquinoline-sulfate, Benthiazole, Bethoxazin, Capsimycin, Carvone, Chinomethionat, Cufraneb, Cyflufenamid, Cymoxanil, Cyprosulfamide, Dazomet, Debacarb, Dichlorophen, Diclomezine, Dicloran, Difenzoquat, Difenzoquat methylsulphate, Diphenylamine, Ferimzone, Flumetover, Fluopicolide, Fluoroimide, Flusulfamide, Fosetyl-Al, Fosetyl-calcium, Fosetyl-natrium, Hexachlorobenzene, Irumamycin, Isotianil, Methasulfocarb, Methyl isothiocyanate, Metrafenone, Mildiomycin, N-(4-Chlor-2-nitrophenyl)-N-ethyl-4-methylbenzenesulfonamide, N-(4-Chlorbenzyl)-3-[3-methoxy-4-(prop-2-yn-1-yloxy) phenyl]propanamide, N-(6-methoxy-3-pyridinyl)-cyclopropanecarboxamide, N-[(4-Chlorphenyl)(cyano)methyl]-3-[3-methoxy-4-(prop-2-yn-1-yloxy)-phenyl]-propanamide, N-[(5-Brom-3-chlorpyridin-2-yl)methyl]-2,4-dichlomicotinamide, N-[1-(5-Brom-3-chlorpyridin-2-yl)ethyl]-2,4-dichlomicotinamide, N-[1-(5-Brom-3-chlorpyridin-2-yl)ethyl]-2-fluoro-4-iodonicotinamide, Natamycin, Nickel-dimethyldithiocarbamate, Nitrothal-isopropyl, Octhilinone, Oxyfenthiin, Pentachlorophenol and salts, Phosphorous acid and its salts, Propamocarb-Fosetyl, Propanosine-sodium, Proquinazid, Pyrrolnitrine, Quintozene, S-allyl-5-amino-2-isopropyl-4-(2-methylphenyl)-3-oxo-2,3-dihydro-1H-pyrazole-1-carbothioate, Tecloftalam, Tecnazene, Triazoxide, Trichlamide, Zarilamid zur Bekämpfung von Fischmykosen bei allen Fischpopulationen und deren Entwicklungsstadien, insbesondere solche, die durch *Saprolegnia* -Erreger hervorgerufen werden.

Fischkrankheiten spielen eine Rolle in der Fischzucht und Aquakultur, in der Aquaristik und in wildlebenden Fischpopulationen. Dabei sind verschiedene Krankheitstypen zu unterscheiden, wie Erbkrankheiten, Infektions- und Parasitenkrankheiten, Verletzungen, wasserbedingten Schäden und Schäden durch Stressfaktoren in den Haltungsbedingungen. Zwischen den Abwehrfähigkeiten, den Krankheitserregern und den Lebensbedingungen herrscht ein komplexer Wirkungszusammenhang, der letztlich über den Ausbruch von Infektionskrankheiten entscheidet. Eine Vielzahl häufiger Fischkrankheiten wird durch den Befall von Parasiten hervorgerufen. Parasiten werden auch zu etwa 50 Prozent für Todesfälle bei Jungtieren in der Aquaristik verantwortlich gemacht. Der Befall mit Parasiten kann, je nach Art des Erregers, schleichend oder explosionsartig erfolgen und dafür sorgen, dass viele bis alle Tiere eines Beckens von Krankheiten betroffen sind. Auch Pilze, Bakterien und Viren können Krankheiten bei Fischen verursachen.

Die durch Pilze verursachten Erkrankungen nennt man Mykosen. Es kann sich dabei auch um Sekundärinfektionen handeln, d.h., dass vor den Mykosen bereits andere Krankheiten den Fisch oder das Becken befallen hatten. Pilze können auch primär parasitär leben. Da Pilze auch über eine wandige Abgrenzung verfügen, sind in die Haut eingedrungene Mykosen nur sehr schwer zu behandeln.

*Saprolegnia* gehört zur Klasse der Oomyceten. Erkrankte Fische zeigen folgende Symptome: sie weisen weiße, wattebauschartige, grauweiße Verpilzungen an ihrer Oberfläche auf. Diese Pilze können sich oftmals dann auf dem Fisch ansiedeln, wenn die schützende Schleimschicht bzw. die Oberhaut verletzt ist. Derartige Pilzwucherungen können die Folge von Stich- oder Bisswunden durch andere Organismen oder von mechanischen Verletzungen sein, aber auch durch Temperatur- oder Abwassereinwirkungen hervorgerufen werden. Gefährdet sind jedoch auch die Fischeier. Der Pilz kommt natürlich vor in allen Süßgewässern und greift geschwächte Fische an. Häufig zeigt es sich, dass besonders ältere männliche Forellen stark betroffen sind. *Saprolegnia* ist sowohl ein Schwächeparasit, der sekundär auftritt, als auch ein Primärparasit, der die Fische und ihre Eier direkt befallen kann. Er kann im Übrigen alle Fischarten befallen.

Ziel der Erfindung ist es, Einsatzgebiete bei der Vermeidung und Behandlung von Schädigungen an Fischen, die bei Abfischung, Transport und Hälterung entstehen, aufzuzeigen, bei der Aufzucht von Fischen sowie bei der Eibehandlung die Ertragslage zu verbessern und einen störungsfreien Betrieb von Anlagen in der Aquakultur und Aquaristik zu gewährleisten.

Die Fungizide können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Die Erfindung umfasst sowohl die reinen Isomeren als auch deren Gemische.

Je nach Art der oben definierten Substituenten weisen die oben aufgeführten Fungizide saure oder basische Eigenschaften auf und können Salze, gegebenenfalls auch innere Salze bilden. Tragen die Fungizide Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkylresten, Mono-, Di- und Trialkanolamine von (C₁-C₄)-Alkanolen, Cholin sowie Chlorcholin. Tragen die Fungizide Amino, Alkylamino oder andere, basische Eigenschaften induzierende Gruppen, so können diese Verbindungen mit Säuren zu Salzen umgesetzt werden. Geeignete Säuren sind beispielsweise Mineralsäuren, wie Salz-, Schwefel- und Phosphorsäure, organische Säuren, wie Essigsäure oder Oxalsäure, und saure Salze, wie NaHSO₄ und KHSO₄. Die so erhältlichen Salze weisen ebenfalls fungizide Eigenschaften auf.

Die erfindungsgemäß zu verwendenen Fungizide sind oben genannt. Beispielsweise aber nicht beschränkend seine folgende Vertreter der einzelnen Wirkstoffgruppen genannt.
1) Inhibitoren der Nukleinsäuresynthese: z.B. Benalaxyl, Benalaxyl-M, Bupirimate, Clozylacon, Dimethirimol, Ethirimol, Furalaxyl, Hymexazol, Mefenoxam, Metalaxyl, Metalaxyl-M, Ofurace, Oxadixyl, Oxolinic acid;
2) Inhibitoren von Mitose und Zellteilung: z.B. Benomyl, Carbendazim, Chlorfenazole, Diethofencarb, Ethaboxam, Fuberidazole, Pencycuron, Profenofos, Thiabendazole, Thiophanate, Thiophanate-methyl, Zoxamide, 5-Chlor-6-(2,4,6-trifluorphenyl)-7-(4-methylpiperidin-1-yl)[1,2,4]triazolo[1,5-a]pyrimidin;
3) Inhibitoren der Respiration (Atmungsketten-Inhibitoren):
   3.1) Inhibitoren am Komplex I der Atmungskette: z.B. Diflumetorim;
   3.2) Inhibitoren am Komplex II der Atmungskette: z.B. 2-Chlor-N-(4'-Prop-1-in-1-ylbiphenyl-2-yl)-Nicotinamid, 2-Chlor-N-[4'-(3,3-Dimethylbut-1-in-1-yl)biphenyl-2-yl]-Nicotinamid, 3-(Difluormethyl)-1-methyl-N-(4'-Prop-1-yn-1-ylbiphenyl-2-yl)-1H-Pyrazol-4-carboxamid, 3-(Difluormethyl)-N-[(9R)-9-Isopropyl-1,2,3,4-Tetrahydro-1,4-methanonaphthalen-5-yl]-1-Methyl-1H-pyrazol-4-carboxamid, 3-(Difluormethyl)-N-[(9S)-9-Isopropyl-1,2,3,4-Tetrahydro-1,4-methano-naphthalen-5-yl]-1-Methyl-1H-pyrazol-4-carboxamid, 3-(Difluormethyl)-N-[2-(3,3-dimethylbutyl)phenyl]-1-methyl-1H-pyrazol-4-carboxamid, 5-Fluor-1,3-Dimethyl-N-(4'-Prop-1-yn-1-ylbiphenyl-2-yl)-1H-Pyrazol-4-carboxamid, N-[2-(1,3-Dimethylbutyl)phenyl]-5-fluor-1,3-dimethyl-1H-pyrazol-4-carboxamid, Bixafen, Boscalid, Carboxin, Fenfuram, Fluopyram, Flutolanil, Furametpyr, Furmecyclox, Mepronil, N-[4'-(3,3-Dimethylbut-1-in-1-yl)biphenyl-2-yl]-3-Difluormethyl-1-Methyl-1H-Pyrazol-4-carboxamid, N-[4'-(3,3-Dimethylbut-1-in-1-yl)biphenyl-2-yl]-5-Fluor-1,3-Dimethyl-1H-Pyrazol-4-carb-oxamid, N-{2-[1,1'-Bi(Cyclopropyl)-2-yl]Phenyl}-3-(Difluormethyl)-1-Methyl-1H-Pyrazol-4-Carboxamid, Oxycarboxin, Penthiopyrad, Thifluzamide;
4) Entkoppler: z.B. Dinocap, Fluazinam, Meptyldinocap;
5) Inhibitoren der ATP Produktion: z.B. Fentin acetate, Fentin chloride, Fentin hydroxide, Silthiofam;
6) Inhibitoren der Aminosäure- und Protein-Biosynthese: z.B. Andoprim, Blasticidin-S, Cyprodinil, Kasugamycin, Kasugamycin hydrochloride hydrate, Mepanipyrim, Pyrimethanil;
7) Inhibitoren der Signaltransduktion: z.B. Fenpiclonil, Fludioxonil, Quinoxyfen;
8) Inhibitoren der Lipid- und Membran-Synthese: z.B. Biphenyl, Chlozolinate, Edifenphos, Etridiazole, lodocarb, Iprobenfos, Iprodione, Isoprothiolane, Procymidone, Propamocarb, Propamocarb hydrochloride, Pyrazophos, Tolclofos-methyl, Vinclozolin;
9) Inhibitoren der Ergosterol-Biosynthese: z.B. Aldimorph, Azaconazole, Bi*tert*anol*,* Bromuconazole, Cyproconazole, Diclobutrazole, Difenoconazole, Diniconazole, Diniconazole-M, Dodemorph, Dodemorph acetate, Epoxiconazole, Etaconazole, Fenarimol, Fenbuconazole, Fenhexamid, Fenpropidin, Fenpropimorph, Fluquinconazole, Flurprimidol, Flusilazole, Flutriafol, Furconazole, Furconazole-cis, Hexaconazole, Imazalil, Imazalil sulfate, Imibenconazole, Ipconazole, Metconazole, Methyl 1-(2,2-dimethyl-2,3-dihydro-1H-inden-1-yl)-1H-imidazole-5-carboxylate, Myclobutanil, Naftifine, N-Ethyl-N-Methyl-N'-{2-Methyl-5-(Difluormethyl)-4-[3-(Trimethylsilyl)propoxy]phenyl}-imido-formamid, N-Ethyl-N-Methyl-N'-{2-Methyl-5-(Trifluormethyl)-4-[3-(Trimethylsilyl)propoxy] phenyl}-imidoformamid, Nuarimol, Oxpoconazole, O-{1-[(4-Methoxyphenoxy)methyl]-2,2-dimethylpropyl}1H-imidazole-1-carbothioate, Paclobutrazol, Pefurazoate, Penconazole, Piperalin, Prochloraz, Propiconazole, Prothioconazole, Pyributicarb, Pyrifenox, Quinconazole, Simeconazole, Spiroxamine, Tebuconazole, Terbinafine, Tetraconazole, Triadimefon, Triadimenol, Tridemorph, Triflumizole, Triforine, Triticonazole, Uniconazole, Viniconazole, Voriconazole, 1-(4-Chlorphenyl)-2-(1H-1,2,4-triazol-1-yl)cycloheptanol;
10) Inhibitoren der Zellwandsynthese: z.B. Benthiavalicarb, Dimethomorph, Flumorph, Iprovalicarb, Mandipropamid, N-[2-(4-{[3-(4-Chlorphenyl)prop-2-yn-1-yl]oxy}-3-methoxyphenyl)ethyl]-N-2-(methylsulfonyl)valinamide, Polyoxins, Polyoxorim, Validamycin A, Valiphenal;
11) Inhibitoren der Melanin-Biosynthese: z.B. Carpropamid, Diclocymet, Fenoxanil, Phthalide, Pyroquilon, Tricyclazole;
12) Resistenzinduktoren: z.B. Acibenzolar-S-methyl, Probenazole, Tiadinil;
13) Verbindungen mit Multisite-Aktivität: z.B. Bordeaux Mixture, Captafol, Captan, Chlorothalonil, Copper hydroxide, Copper naphthenate, Copper oxide, Copper oxychloride, Copper sulphate, Dichlofluanid, Dithianon, Dodine, Dodine free base, Ferbam, Fluorofolpet, Folpet, Guazatine, Guazatine acetate, Iminoctadine, Iminoctadine albesilate, Iminoctadine triacetate, Mancopper, Mancozeb, Maneb, Metiram, Metiram Zinc, Oxine-Copper, Propineb, Sulphur und Schwefelzubereitungen wie z.B. Calcium polysulphide, Thiram, Tolylfluanid, Zineb, Ziram;
14) eine Verbindung aus der folgenden Liste: 2,3,5,6-Tetrachlor-4-(methylsulfonyl)pyridine, 2,3-Dibutyl-6-chloro-thieno[2,3-d]pyrimidin-4(3H)one, 2-Butoxy-6-iod-3-propyl-benzopyran-4-on, 2-chloro-N-(2,3-dihydro-1,1,3-trimethyl-1H-inden-4-yl)-3-pyridincarboxamide, 3,4,5-Trichlor-pyridine-2,6-dicarbonitrile, 3-[5-(4-Chlorphenyl)-2,3-dimethylisoxazolidin-3-yl]pyridine, 8-hydroxyquinoline-sulfate, Benthiazole, Bethoxazin, Capsimycin, Carvone, Chinomethionat, Cufraneb, Cyflufenamid, Cymoxanil, Cyprosulfamide, Dazomet, Debacarb, Dichlorophen, Diclomezine, Dicloran, Difenzoquat, Difenzoquat methylsulphate, Diphenylamine, Ferimzone, Flumetover, Fluopicolide, Fluoroimide, Flusulfamide, Fosetyl-A1, Fosetyl-calcium, Fosetyl-natrium, Hexachlorobenzene, Irumamycin, Isotianil, Methasulfocarb, Methyl isothiocyanate, Metrafenone, Mildiomycin, N-(4-Chlor-2-nitrophenyl)-N-ethyl-4-methylbenzenesulfonamide, N-(4-Chlorbenzyl)-3-[3-methoxy-4-(prop-2-yn-1-yloxy)phenyl]propanamide, N-(6-methoxy-3-pyridinyl)-cyclopropanecarboxamide, N-[(4-Chlorphenyl)(cyano)methyl]-3-[3-methoxy-4-(prop-2-yn-1-yloxy)-phenyl]-propanamide, N-[(5-Brom-3-chlorpyridin-2-yl)methyl]-2,4-dichlornico-tinamide, N-[1-(5-Brom-3-chlorpyridin-2-yl)ethyl]-2,4-dichlomicotinamide, N-[1-(5-Brom-3-chlorpyridin-2-yl)ethyl]-2-fluoro-4-iodonicotinamide, Natamycin, Nickeldimethyldithiocarbamate, Nitrothal-isopropyl, Octhilinone, Oxyfenthiin, Pentachlorophenol and salts, Phosphorous acid and its salts, Propamocarb-Fosetyl, Propanosine-sodium, Proquinazid, Pyrrolnitrine, Quintozene, S-allyl-5-amino-2-isopropyl-4-(2-methylphenyl)-3-oxo-2,3-dihydro-1H-pyrazole-1-carbothioate, Tecloftalam, Tecnazene, Triazoxide, Trichlamide, Zarilamid.

Besonders bevorzugt sind folgende Vertreter der folgenden Wirkstoffgruppen:
1) Inhibitoren der Nukleinsäuresynthese: Benalaxyl, Metalaxyl, Metalaxyl-M, Ofurace, Oxadixyl;
2) Inhibitoren von Mitose und Zellteilung: Ethaboxam, Zoxamide, 5-Chlor-6-(2,4,6-trifluorphenyl)-7-(4-methylpiperidin-1-yl)[1,2,4]triazolo[1,5-a]pyrimidin;
3) Inhibitoren der Respiration (Atmungsketten-Inhibitoren):
   3.2) Inhibitoren am Komplex II der Atmungskette: 3-(Difluormethyl)-N-[(9R)-9-Isopropyl-1,2,3,4-Tetrahydro-1,4-methanonaphthalen-5-yl]-1-Methyl-1H-pyrazol-4-carboxamid, 3-(Difluormethyl)-N-[(9S)-9-Isopropyl-1,2,3,4-Tetrahydro-1,4-methanonaphthalen-5-yl]-1-Methyl-1H-pyrazol-4-carboxamid, N-[2-(1,3-Dimethylbutyl)phenyl]-5-fluor-1,3-dimethyl-1H-pyrazol-4-carboxamid, Bixafen, Boscalid, Fluopyram, N-{2-[1,1'-Bi(Cyclopropyl)-2-yl]Phenyl}-3-(Difluormethyl)-1-Methyl-1H-Pyrazol-4-Carboxamid;
4) Entkoppler: Fluazinam;
6) Inhibitoren der Aminosäure- und Protein-Biosynthese: Pyrimethanil;
8) Inhibitoren der Lipid- und Membran-Synthese: Iprodione, Propamocarb, Propamocarb hydrochloride;
9) Inhibitoren der Ergosterol-Biosynthese: Bitertanol, Cyproconazole, Difenoconazole, Epoxiconazole, Flusilazole, Prochloraz, Propiconazole, Prothioconazole, Spiroxamine, Tebuconazole, Triadimefon, Triadimenol;
10) Inhibitoren der Zellwandsynthese: Benthiavalicarb, Dimethomorph, Iprovalicarb, Mandipropamid;
13) Verbindungen mit Multisite-Aktivität: Captan, Chlorothalonil, Folpet, Mancozeb, Maneb, Propineb, Tolylfluanid;
14) eine Verbindung aus der folgenden Liste: Cymoxanil, Fluopicolide, Fluoxastrobin, Fosetyl-Al, Isotianil, N-(6-methoxy-3-pyridinyl)-cyclopropanecarboxamide;

Ganz besonders bevorzugt sind folgende Inhibitoren der Nukleinsäuresynthese: Benalaxyl, Metalaxyl-M.

Weiterhin ganz besonders bevorzugt sind folgende Inhibitoren von Mitose und Zellteilung: Ethaboxam, Zoxamide.

Weiterhin ganz besonders bevorzugt sind folgende Inhibitoren am Komplex II der Atmungskette: N-[2-(1,3-Dimethylbutyl)phenyl]-5-fluor-1,3-dimethyl-1H-pyrazol-4-carboxamid, Bixafen, Boscalid, Fluopyram.

Weiterhin ganz besonders bevorzugt sind folgende Entkoppler: Fluazinam.

Weiterhin ganz besonders bevorzugt sind folgende Inhibitoren der Aminosäure- und Protein-Biosynthese: Pyrimethanil.

Weiterhin ganz besonders bevorzugt sind folgende Inhibitoren der Lipid- und Membran-Synthese: Iprodione, Propamocarb, Propamocarb hydrochloride.

Weiterhin ganz besonders bevorzugt sind folgende Inhibitoren der Ergosterol-Biosynthese: Cyproconazole, Difenoconazole, Epoxiconazole, Prochloraz, Propiconazole, Prothioconazole, Spiroxamine, Tebuconazole.

Weiterhin ganz besonders bevorzugt sind folgende Inhibitoren der Zellwandsynthese: Benthiavalicarb, Dimethomorph, Iprovalicarb, Mandipropamid.

Weiterhin ganz besonders bevorzugt sind folgende Verbindungen mit Multisite-Aktivität: Captan, Chlorothalonil, Folpet, Mancozeb, Maneb, Propineb, Tolylfluanid.

Weiterhin ganz besonders bevorzugt sind folgende Verbindungen aus der folgenden Liste: Cymoxanil, Fluopicolide, Fosetyl-Al, Isotianil.

Insbesondere bevorzugt sind Verbindungen ausgewählt aus folgender Gruppe:

Benthiavalicarb, Bixafen, Dimetomorph, Epoxiconazole, Ethaboxam, Fluopicolide, Fluopyram, Fosetyl-A1, Iprovalicarb, Isotianil, Mandipropamid, N-[2-(1,3-Dimethylbutyl)phenyl]-5-fluor-1,3-dimethyl-1H-pyrazol-4-carboxamid, 5-Chlor-6-(2,4,6-trifluorphenyl)-7-(4-methylpiperidin-1-yl)-[1,2,4]triazolo[1,5-a]pyrimidin, Propamocarb, Propamocarb hydrochloride, Prothioconazole, Pyrimethanil, Tebuconazole und Zoxamide.

In den erfindungsgemäßen Verfahren, Verwendungen und Mitteln können sowohl einzelne oder mehrere Fungizide in Kombination ausgewählt sein.

Erfindungsgemäss werden die Fungizide dem Wasser von Aquakulturanlagen z.B. in Erbrütungsanlagen, Zuchtteichen, Zuchttanks, Rundschwimmbecken, Mastbecken, Aquarien, natürlichen Sportfischgewässern und marinen Fischzuchten zugesetzt und durch ihre Einwirkung die Wachstumshemmung, bzw. Abtötung pathogener Pilze bewirkt. Aquakulturanlagen in diesem Sinne sind Einrichtungen, die der Aufzucht von Fischen oder Wirbellosen in Süß-, Brack- oder Salzwasser dienen. Die Fungizide werden dem Wasser zugesetzt. Die Dosis richtet sich nach der Beschaffenheit (organische Belastung) des Wassers in der Aquakulturanlage, der Aktivität des Fungizdes und dem zu behandelnden Entwicklungsstadium der Fische. Der Aktivitätsspiegel wird durch kontinuierliche oder diskontinuierliche Zugabe aufrechterhalten, was bestehende Mykosen zurückdrängt und das Auftreten von Neuinfektionen verhindert.

Die Aufwandmengen liegen, je nach Art und Entwicklungsstadium der Fische und nach Art des Fungizids, bei 0,1µg/l bis 1g/l, vorzugsweise 1µg/l bis 100mg/l, insbesondere bevorzugt 5µg/l bis 1mg/l an Wirkstoff. Höhere Konzentrationen sind im Allgemeinen nicht erforderlich, können aber je nach Art der Verbindung oder Anwendung besonders in künstlichen Systemen, wie z.B. Zuchttanks oder Aquarien, bei der Behandlung von Ei-, Larven- und Jungstadien nützlich sein.

Eine Wirkung gegen Mykosen wird weiterhin dadurch erzielt, dass die Fungizide nicht nur frei im wässrigen Milieu der Aquakulturanlagen eingesetzt werden, sondern auch an die Oberfläche (Schleimhaut) zu schützender Organismen oder deren Eier gebunden werden. Diese Vorgehensweise ist dann vorzuziehen, wenn eine hohe Infektionsgefahr einen höheren Fungizidgehalt erforderlich macht oder bei hohem Wasserdurchsatz der Aquakulturanlage ein ständiges Zudosieren von Fungizidgemisch nicht möglich ist. Die Bindung wird durch Behandlung der Tiere oder deren Eier durch eine zeitlich begrenzte Vorinkubation bei erhöhten Fungizidkonzentrationen erreicht, wobei neben dem Fungizidgemisch vorzugsweise Stoffe eingesetzt werden, welche eine Bindung der Fungizide verstärken.

Die wirksame Menge eines Fungizids umfasst beispielhafte Dosierungsmengen für einen Fisch von etwa 1µg bis 10 mg/kg/Tag, die in einer Einzeldosis oder in der Form von einzelnen aufgeteilten Dosen, wie 1 bis 4 mal pro Tag, verabreicht werden können. Vorzugsweise werden die Verbindungen in einer Dosierung von weniger als 10 mg/kg/Tag, in einer Einzeldosis oder in 2 bis 4 aufgeteilten Dosen verabreicht.

Die Wirkstoffe können direkt angewendet werden oder vor der Anwendung mit üblichen inerten Trägerstoffen gemischt werden. Grundsätzlich eignen sich als inerte Trägerstoffe solche Substanzen, die eine homogene Verteilung des Wirkstoffs im Wasser beziehungsweise an der Oberfläche (Schleimhaut) zu schützender Organismen oder deren Eiern erleichtern bzw. gewährleisten.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol, sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel, wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe, wie anorganische Pigmente, z.B. Eisenoxid, Titanoxid, Ferrocyanblau, und organische Farbstoffe, wie Alizarin-, Azo-und Metallphthalocyaninfarbstoffe, und Spurennährstoffe, wie Salze von Eisen, Mangan, Bor, Kupfer, Kobalt, Molybdän und Zink, verwendet werden.

Die Formulierungen enthalten im Allgemeinen zwischen 0,1 und 95 Gewichtsprozent Wirkstoff, vorzugsweise zwischen 0,5 und 90 %.

Gegenstand der Erfindung ist ein Verfahren zur Behandlung von an Mykosen erkrankten Fischen und all ihren Entwicklungsstadien mit wirksamen Fungiziden.

Es ist ersichtlich, dass die spezifische Dosis und die Häufigkeit der Dosierung für bestimmte Fischarten und je nach Entwicklungsstadium variiert werden können und von einer Vielzahl von Faktoren abhängen, einschließlich der Wirksamkeit der verwendeten spezifischen Verbindung, der metabolischen Stabilität und der Länge der Wirkung dieser Verbindung, der Spezies, dem Alter, dem Gewicht, dem allgemeinen Gesundheitszustand, dem Geschlecht und der Ernährung der Fische, der Art und der Zeit der Verabreichung, der Ausscheidungsrate und der Schwere des besonderen Zustands.

Die vorliegende Erfindung stellt also ein Veterinärarzneimittel bereit, das mindestens ein Fungizid umfasst, mit der *Saprolegnia-* Erkrankungen oder andere Fischkrankheiten behandelt werden können, in einer dafür wirksamen Menge und einen pharmazeutisch verträglichen Träger oder ein pharmazeutisch verträgliches Verdünnungsmittel. Die erfindungsgemäßen Zusammensetzungen können andere therapeutische Mittel, wie nachstehend beschrieben, enthalten, und können beispielsweise unter Verwendung herkömmlicher fester oder flüssiger Träger oder Verdünnungsmittel, wie pharmazeutischer Zusätze eines Typs, der für die gewünschten Verabreichung geeignet ist (beispielsweise Excipienten, Bindemittel, Konservierungsmittel, Stabilisatoren, Aromastoffe usw.) gemäß Techniken, die auf dem Gebiet der pharmazeutischen Galenik wohlbekannt sind oder durch akzeptierte pharmazeutische Praxis gefordert werden, formuliert werden.

Die oben beschriebenen wirksamen Fungizide können durch ein beliebiges geeignetes Mittel, beispielsweise oral, wie in der Form von Tabletten, Kapseln, Körnchen oder Pulver, sublingual, bukkal, parenteral, wie mittels subkutaner, intravenöser, intramuskulärer oder intrasternaler Injektions- oder Infusionstechniken (z.B. als sterile, injizierbare, wässerige oder nichtwässerige Lösungen oder Suspensionen), topisch, wie in der Form einer Creme oder Salbe oder in Dosierungseinheitsformulierungen, die nichttoxische, pharmazeutisch verträgliche Träger oder Verdünnungsmittel enthalten, verabreicht werden. Die Fungizide können beispielsweise in einer Form verabreicht werden, die für eine sofortige Freisetzung oder verlängerte Freisetzung geeignet ist. Die sofortige Freisetzung oder die verlängerte Freisetzung können durch die Verwendung geeigneter Arzneimittel erzielt werden, die die oben beschriebenen wirksamen Fungizide enthalten oder insbesondere im Fall der verlängerten Freisetzung durch die Verwendung von Vorrichtungen, wie subkutanen Implantaten oder osmotischen Pumpen. Die Verbindungen können auch liposomal verabreicht werden. Beispielsweise kann die Wirksubstanz in einer Zusammensetzung wie einer Tablette, einer Kapsel, einer Lösung oder Suspension verwendet werden, die etwa 5 bis etwa 500 mg pro Einheitsdosierung einer Verbindung oder Mischung von Verbindungen aus der Liste der oben genannten Fungizide oder in einer topischen Form (0,01 bis 5 Gew.-% Fungizid, ein bis fünf Behandlungen pro Tag) verwendet werden. Sie kann auf herkömmliche Weise mit einem physiologisch verträglichen Träger, Excipienten, Bindemittel, Konservierungsmittel, Stabilisator, Aromastoff usw. oder mit einem topischen Träger vermischt werden. Die Verbindungen können auch in Zusammensetzungen wie sterilen Lösungen oder Suspensionen für die parenterale Verabreichung formuliert werden. Etwa 0,1 bis 500 mg eines Fungizids kann mit einem physiologisch verträglichen Träger, Excipientien, Bindemittel, Konservierungsmittel, Stabilisator usw. in einer Einheitsdosierungsform vermischt werden, wie dies durch die akzeptierte pharmazeutische Praxis gefordert wird. Die Menge der Wirksubstanz in diesen Zusammensetzungen oder Zubereitungen ist vorzugsweise eine solche, dass eine geeignete Dosierung in dem angegebenen Bereich erhalten wird.

Beispielhafte Zusammensetzungen für die orale Verabreichung umfassen Suspensionen, die beispielsweise mikrokristalline Cellulose zur Verleihung von Masse, Alginsäure oder Natriumalginat als Suspensionsmittel, Methylcellulose als Viskositätsverstärker und Süßungsmittel oder Aromastoffe, wie diejenigen, die in der Technik bekannt sind, enthalten können, und Tabletten mit sofortiger Freisetzung, die beispielsweise mikrokristalline Cellulose, Dicalciumphosphat, Stärke, Magnesiumstearat und/oder Lactose und/oder andere Excipientien, Bindemittel, Streckmittel, Abbaumittel, Verdünnungsmittel und Gleitmittel, wie diejenigen, die in der Technik bekannt sind, enthalten können. Formtabletten, Presstabletten oder gefriergetrocknete Tabletten sind beispielhafte Formen, die verwendet werden können. Beispielhafte Zusammensetzungen umfassen diejenigen, die die fungiziden Wirkstoffe mit schnell löslichen Lösungsmitteln wie Mannitol, Lactose, Sucrose und/oder Cyclodextrinen formulieren. In solchen Formulierungen können auch Excipientien mit hohem Molekulargewicht wie Cellulosen (Avicel) oder Polyethylenglycole (PEG) enthalten sein. Solche Formulierungen können auch einen Excipientien, um die Schleimhautadhäsion zu unterstützen, wie Hydroxypropylcellulose (HPC), Hydroxypropylmethylcellulose (HPMC), Natriumcarboxymethylcellulose (SCMC), Maleinsäureanhydrid-Copolymer (z.B. Gantrez) und Mittel zur Steuerung der Freisetzung wie Polyacryl-Copolymer (z.B. Carbopol 934), enthalten. Schmiermittel, Gleitmittel, Aromastoffe, Färbemittel und Stabilisatoren können auch für eine leichtere Herstellung und Verwendung zugegeben werden.

Die oben erwähnten Fungizide können entweder allein oder in Kombination mit anderen weiteren Fungiziden verabreicht werden. Insbesondere nützlich sind Kombinationen aus Fungiziden, die ebenfalls eine Wirkung gegen Fischmykosen aufweisen, um die Wirkung entsprechend abzusichern und wirksam die Entstehung einer Resistenz des pilzlichen Erregers gegen den Wirkstoff zu verhindern.

Die Erfindung wird durch folgendes Beispiel näher erläutert.

### Ausfiihrungsbeispiel

### Beispiel 1: Ermittlung der Wirksamkeit von Substanzen gegen fischparasitäre Pilze, zum Beispiel Saprolegnia spp.

Ein Isolat von *Saprolegnia parasitica* (CBS 540.67, Centraalbureau voor Schimmelcultures, Baam, Niederlande) wird auf PD-Agar (39 g/ 1 Endkonzentration) bei 20° C im Dunkeln angezogen und vermehrt. Zur Bestimmung des ED₅₀-Wertes werden PD-Agarplatten mit einer Konzentrationsreihe von 0 - 0.03 - 0.1 - 0.3 - 1 - 3 - 10 - 30 ppm des zu prüfenden Fungizides versetzt. Jeweils in die Mitte der mit den verschiedenen Konzentrationen des Fungizides versetzten Petrischalen werden Impfstücke mit *Saprolegnia parasitica* platziert.

Die Platten werden für drei Tage bei 20° C im Dunkeln inkubiert und danach wird das Wachstum des Pilzes auf der Agarplatte vermessen und die ED₅₀-Werte aus dem Vergleich zur unbehandelten Kontrolle kalkuliert.

In diesem Test zeigen beispielsweise die Fungizide Fluopicolide und Tebuconazole einen ED₅₀-Wert von 5 ppm oder kleiner.

## Patentansprüche

1. Mittel zur Behandlung von Mykosen bei Fischen und Wirbellosen und all deren Entwicklungsstadien, hervorgerufen durch Pilze der Gattungen *Saprolegnia, Achlya, Aphanomyces* **gekennzeichnet durch** einen Gehalt an mindestens einem Fungizid ausgewählt aus folgender Gruppe: 1) Inhibitoren der Nukleinsäuresynthese: z.B. Benalaxyl, Benalaxyl-M, Bupirimate, Clozylacon, Dimethirimol, Ethirimol, Furalaxyl, Hymexazol, Mefenoxam, Metalaxyl, Metalaxyl-M, Ofurace, Oxadixyl, Oxolinic acid;
2) Inhibitoren von Mitose und Zellteilung: z.B. Benomyl, Carbendazim, Chlorfenazole, Diethofencarb, Ethaboxam, Fuberidazole, Pencycuron, Profenofos, Thiabendazole, Thiophanate, Thiophanate-methyl, Zoxamide, 5-Chlor-6-(2,4,6-trifluorphenyl)-7-(4-methylpiperidin-1-yl)[1,2,4]triazolo[1,5-a]pyrimidin;
3) Inhibitoren der Respiration (Atmungsketten-Inhibitoren):
3.1) Inhibitoren am Komplex I der Atmungskette: z.B. Diflumetorim;
3.2) Inhibitoren am Komplex II der Atmungskette: z.B. 2-Chlor-N-(4'-Prop-1-in-1-ylbiphenyl-2-yl)-Nicotinamid, 2-Chlor-N-[4'-(3,3-Dimethylbut-1-in-1-yl)biphenyl-2-yl]-Nicotinamid, 3-(Difluormethyl)-1-methyl-N-(4'-Prop-1-yn-1-ylbiphenyl-2-yl)-1H-Pyrazol-4-carboxamid, 3-(Difluormethyl)-N-[(9R)-9-Isopropyl-1,2,3,4-Tetrahydro-1,4-methano-naphthalen-5-yl]-1-Methyl-1H-pyrazol-4-carboxamid, 3-(Difluormethyl)-N-[(9S)-9-Isopropyl-1,2,3,4-Tetrahydro-1,4-methanonaphthalen-5-yl]-1-Methyl-1H-pyrazol-4-carboxamid, 3-(Difluormethyl)-N-[2-(3,3-dimethylbutyl)phenyl]-1-methyl-1H-pyrazol-4-carboxamid, 5-Fluor-1,3-Dimethyl-N-(4'-Prop-1-yn-1-ylbiphenyl-2-yl)-1H-Pyrazol-4-carboxamid, N-[2-(1,3-Dimethylbutyl)phenyl]-5-fluor-1,3-dimethyl-1H-pyrazol-4-carboxamid, Bixafen, Boscalid, Carboxin, Fenfuram, Fluopyram, Flutolanil, Furametpyr, Furmecyclox, Mepronil, N-[4'-(3,3-Dimethylbut-1-in-1-yl)biphenyl-2-yl]-3-Difluormethyl-1-Methyl-1H-Pyrazol-4-carboxamid, N-[4'-(3,3-Dimethylbut-1-in-1-yl)biphenyl-2-yl]-5-Fluor-1,3-Dimethyl-1H-Pyrazol-4-carb-oxamid, N-{2-[1,1'-Bi(Cyclopropyl)-2-yl]Phenyl}-3-(Difluormethyl)-1-Methyl-1H-Pyrazol-4-Carboxamid, Oxycarboxin, Penthiopyrad, Thifluzamide;
4) Entkoppler: z.B. Dinocap, Fluazinam, Meptyldinocap;
5) Inhibitoren der ATP Produktion: z.B. Fentin acetate, Fentin chloride, Fentin hydroxide, Silthiofam;
6) Inhibitoren der Aminosäure- und Protein-Biosynthese: z.B. Andoprim, Blasticidin-S, Cyprodinil, Kasugamycin, Kasugamycin hydrochloride hydrate, Mepanipyrim, Pyrimethanil;
7) Inhibitoren der Signaltransduktion: z.B. Fenpiclonil, Fludioxonil, Quinoxyfen;
8) Inhibitoren der Lipid- und Membran-Synthese: z.B. Biphenyl, Chlozolinate, Edifenphos, Etridiazole, Iodocarb, Iprobenfos, Iprodione, Isoprothiolane, Procymidone, Propamocarb, Propamocarb hydrochloride, Pyrazophos, Tolclofos-methyl, Vinclozolin;
9) Inhibitoren der Ergosterol-Biosynthese: z.B. Aldimorph, Azaconazole, Bi*tert*anol, Bromuconazole, Cyproconazole, Diclobutrazole, Difenoconazole, Diniconazole, Diniconazole-M, Dodemorph, Dodemorph acetate, Epoxiconazole, Etaconazole, Fenarimol, Fenbuconazole, Fenhexamid, Fenpropidin, Fenpropimorph, Fluquinconazole, Flurprimidol, Flusilazole, Flutriafol, Furconazole, Furconazole-cis, Hexaconazole, Imazalil, Imazalil sulfate, Imibenconazole, Ipconazole, Metconazole, Methyl 1-(2,2-dimethyl-2,3-dihydro-1H-inden-1-yl)-1H-imidazole-5-carboxylate, Myclobutanil, Naftifine, N-Ethyl-N-Methyl-N'-{2-Methyl-5-(Difluormethyl)-4-[3-(Trimethylsilyl)propoxy]phenyl}-imidoformamid, N-Ethyl-N-Methyl-N'-{2-Methyl-5-(Trifluormethyl)-4-[3-(Trimethylsilyl)propoxy]phenyl}-imido-formamid, Nuarimol, Oxpoconazole, O-{1-[(4-Methoxyphenoxy)methyl]-2,2-dimethylpropyl} 1H-imidazole-1-carbothioate, Paclobutrazol, Pefurazoate, Penconazole, Piperalin, Prochloraz, Propiconazole, Prothioconazole, Pyributicarb, Pyrifenox, Quinconazole, Simeconazole, Spiroxamine, Tebuconazole, Terbinafine, Tetraconazole, Triadimefon, Triadimenol, Tridemorph, Triflumizole, Triforine, Triticonazole, Uniconazole, Viniconazole, Voriconazole, 1-(4-Chlorphenyl)-2-(1H-1,2,4-triazol-1-yl)cycloheptanol;
10) Inhibitoren der Zellwandsynthese: z.B. Benthiavalicarb, Dimethomorph, Flumorph, Iprovalicarb, Mandipropamid, N-[2-(4-{[3-(4-Chlorphenyl)prop-2-yn-l-yl]oxy}-3-methoxyphenyl)ethyl]-N-2-(methylsulfonyl)valinamide, Polyoxins, Polyoxorim, Validamycin A, Valiphenal;
11) Inhibitoren der Melanin-Biosynthese: z.B. Carpropamid, Diclocymet, Fenoxanil, Phthalide, Pyroquilon, Tricyclazole;
12) Resistenzinduktoren: z.B. Acibenzolar-S-methyl, Probenazole, Tiadinil;
13) Verbindungen mit Multisite-Aktivität: z.B. Bordeaux Mixture, Captafol, Captan, Chlorothalonil, Copper hydroxide, Copper naphthenate, Copper oxide, Copper oxychloride, Copper sulphate, Dichlofluanid, Dithianon, Dodine, Dodine free base, Ferbam, Fluorofolpet, Folpet, Guazatine, Guazatine acetate, Iminoctadine, Iminoctadine albesilate, Iminoctadine triacetate, Mancopper, Mancozeb, Maneb, Metiram, Metiram Zinc, Oxine-Copper, Propineb, Sulphur und Schwefelzubereitungen wie z.B. Calcium polysulphide, Thiram, Tolylfluanid, Zineb, Ziram;
14) eine Verbindung aus der folgenden Liste: 2,3,5,6-Tetrachlor-4-(methylsulfonyl)pyridine, 2,3-Dibutyl-6-chloro-thieno[2,3-d]pyrimidin-4(3H)one, 2-Butoxy-6-iod-3-propyl-benzo-pyran-4-on, 2-chloro-N-(2,3-dihydro-1,1,3-trimethyl-1H-inden-4-yl)-3-pyridincarboxamide, 3,4,5-Trichlorpyridine-2,6-dicarbonitrile, 3-[5-(4-Chlorphenyl)-2,3-dimethyl-isoxazolidin-3-yl]pyridine, 8-hydroxyquinoline-sulfate, Benthiazole, Bethoxazin, Capsimycin, Carvone, Chinomethionat, Cufraneb, Cyflufenamid, Cymoxanil, Cyprosulfamide, Dazomet, Debacarb, Dichlorophen, Diclomezine, Dicloran, Difenzoquat, Difenzoquat methylsulphate, Diphenylamine, Ferimzone, Flumetover, Fluopicolide, Fluoroimide, Flusulfamide, Fosetyl-A1, Fosetyl-calcium, Fosetyl-natrium, Hexachlorobenzene, Irumamycin, Isotianil, Methasulfocarb, Methyl isothiocyanate, Metrafenone, Mildiomycin, N-(4-Chlor-2-nitrophenyl)-N-ethyl-4-methylbenzene-sulfonamide, N-(4-Chlorbenzyl)-3-[3-methoxy-4-(prop-2-yn-1-yloxy)phenyl]propanamide, N-(6-methoxy-3-pyridinyl)-cyclopropanecarboxamide, N-[(4-Chlorphenyl)(cyano)methyl]-3-[3-methoxy-4-(prop-2-yn-1-yloxy)-phenyl]-propanamide, N-[(5-Brom-3-chlorpyridin-2-yl)methyl]-2,4-dichlomicotinamide, N-[1-(5-Brom-3-chlorpyridin-2-yl)ethyl]-2,4-dichlomicotinamide, N-[1-(5-Brom-3-chlorpyridin-2-yl)ethyl]-2-fluoro-4-iodonicotinamide, Natamycin, Nickel-dimethyldithiocarbamate, Nitrothal-isopropyl, Octhilinone, Oxyfenthiin, Pentachlorophenol and salts, Phosphorous acid and its salts, Propamocarb-Fosetyl, Propanosine-sodium, Proquinazid, Pyrrolnitrine, Quintozene, S-allyl-5-amino-2-isopropyl-4-(2-methylphenyl)-3-oxo-2,3-dihydro-1H-pyrazole-1-carbothioate, Tecloftalam, Tecnazene, Triazoxide, Trichlamide, Zarilamid.

2. Mittel nach Anspruch 1 **gekennzeichnet durch** einen Gehalt an mindestens einem Fungizid ausgewählt aus folgender Gruppe: Benthiavalicarb, Bixafen, Dimetomorph, Epoxiconazole, Ethaboxam, Fluopicolide, Fluopyram, Fosetyl-A1, Iprovalicarb, Isotianil, Mandipropamid, N-[2-(1,3-Dimethylbutyl)phenyl]-5-fluor-1,3-dimethyl-1H-pyrazol-4-carboxamid, 5-Chlor-6-(2,4,6-trifluorphenyl)-7-(4-methylpiperidin-1-yl)[1,2,4]triazolo-[1,5-a]pyrimidin, Propamocarb, Propamocarb hydrochloride, Prothioconazole, Pyrimethanil, Tebuconazole und Zoxamide.

3. Verwendung von mindestens einem Fungizid ausgewählt aus folgender Gruppe 1) Inhibitoren der Nukleinsäuresynthese: z.B. Benalaxyl, Benalaxyl-M, Bupirimate, Clozylacon, Dimethirimol, Ethirimol, Furalaxyl, Hymexazol, Mefenoxam, Metalaxyl, Metalaxyl-M, Ofurace, Oxadixyl, Oxolinic acid;
2) Inhibitoren von Mitose und Zellteilung: z.B. Benomyl, Carbendazim, Chlorfenazole, Diethofencarb, Ethaboxam, Fuberidazole, Pencycuron, Profenofos, Thiabendazole, Thiophanate, Thiophanate-methyl, Zoxamide, 5-Chlor-6-(2,4,6-trifluorphenyl)-7-(4-methylpiperidin-1-yl)[1,2,4]triazolo[1,5-a]pyrimidin;
3) Inhibitoren der Respiration (Atmungsketten-Inhibitoren):
3.1) Inhibitoren am Komplex I der Atmungskette: z.B. Diflumetorim;
3.2) Inhibitoren am Komplex II der Atmungskette: z.B. 2-Chlor-N-(4'-Prop-1-in-1-ylbiphenyl-2-yl)-Nicotinamid, 2-Chlor-N-[4'-(3,3-Dimethylbut-1-in-1-yl)biphenyl-2-yl]-Nicotinamid, 3-(Difluormethyl)-1-methyl-N-(4'-Prop-1-yn-1-ylbiphenyl-2-yl)-1H-Pyrazol-4-carboxamid, 3-(Difluormethyl)-N-[(9R)-9-Isopropyl-1,2,3,4-Tetrahydro-1,4-methano-naphthalen-5-yl]-1-Methyl-1H-pyrazol-4-carboxamid, 3-(Difluormethyl)-N-[(9S)-9-Isopropyl-1,2,3,4-Tetrahydro-1,4-methanonaphthalen-5-yl]-1-Methyl-1H-pyrazol-4-carboxamid, 3-(Difluormethyl)-N-[2-(3,3-dimethylbutyl)phenyl]-1-methyl-1H-pyrazol-4-carboxamid, 5-Fluor-1,3-Dimethyl-N-(4'-Prop-1-yn-1-ylbiphenyl-2-yl)-1H-Pyrazol-4-carboxamid, N-[2-(1,3-Dimethylbutyl)phenyl]-5-fluor-1,3-dimethyl-1H-pyrazol-4-carboxamid, Bixafen, Boscalid, Carboxin, Fenfuram, Fluopyram, Flutolanil, Furametpyr, Furmecyclox, Mepronil, N-[4'-(3,3-Dimethylbut-1-in-1-yl)biphenyl-2-yl]-3-Difluormethyl-1-Methyl-1H-Pyrazol-4-carboxamid, N-[4'-(3,3-Dimethylbut-1-in-1-yl)biphenyl-2-yl]-5-Fluor-1,3-Dimethyl-1H-Pyrazol-4-carb-oxamid, N-{2-[1,1'-Bi(Cyclopropyl)-2-yl]Phenyl}-3-(Difluormethyl)-1-Methyl-1H-Pyrazol-4-Carboxamid, Oxycarboxin, Penthiopyrad, Thifluzamide;
4) Entkoppler: z.B. Dinocap, Fluazinam, Meptyldinocap;
5) Inhibitoren der ATP Produktion: z.B. Fentin acetate, Fentin chloride, Fentin hydroxide, Silthiofam;
6) Inhibitoren der Aminosäure- und Protein-Biosynthese: z.B. Andoprim, Blasticidin-S, Cyprodinil, Kasugamycin, Kasugamycin hydrochloride hydrate, Mepanipyrim, Pyrimethanil;
7) Inhibitoren der Signaltransduktion: z.B. Fenpiclonil, Fludioxonil, Quinoxyfen;
8) Inhibitoren der Lipid- und Membran-Synthese: z.B. Biphenyl, Chlozolinate, Edifenphos, Etridiazole, Iodocarb, Iprobenfos, Iprodione, Isoprothiolane, Procymidone, Propamocarb, Propamocarb hydrochloride, Pyrazophos, Tolclofos-methyl, Vinclozolin;
9) Inhibitoren der Ergosterol-Biosynthese: z.B. Aldimorph, Azaconazole, Bi*tert*anol, Bromuconazole, Cyproconazole, Diclobutrazole, Difenoconazole, Diniconazole, Diniconazole-M, Dodemorph, Dodemorph acetate, Epoxiconazole, Etaconazole, Fenarimol, Fenbuconazole, Fenhexamid, Fenpropidin, Fenpropimorph, Fluquinconazole, Flurprimidol, Flusilazole, Flutriafol, Furconazole, Furconazole-cis, Hexaconazole, Imazalil, Imazalil sulfate, Imibenconazole, Ipconazole, Metconazole, Methyl 1-(2,2-dimethyl-2,3-dihydro-1H-inden-1-yl)-1H-imidazole-5-carboxylate, Myclobutanil, Naftifine, N-Ethyl-N-Methyl-N'-{2-Methyl-5-(Difluormethyl)-4-[3-(Trimethylsilyl)propoxy]phenyl}-imidoformamid, N-Ethyl-N-Methyl-N'-{2-Methyl-5-(Trifluormethyl)-4-[3-(Trimethylsilyl)propoxy]phenyl}-imido-formamid, Nuarimol, Oxpoconazole, O-{1-[(4-Methoxyphenoxy)methyl]-2,2-dimethylpropyl} 1H-imidazole-1-carbothioate, Paclobutrazol, Pefurazoate, Penconazole, Piperalin, Prochloraz, Propiconazole, Prothioconazole, Pyributicarb, Pyrifenox, Quinconazole, Simeconazole, Spiroxamine, Tebuconazole, Terbinafine, Tetraconazole, Triadimefon, Triadimenol, Tridemorph, Triflumizole, Triforine, Triticonazole, Uniconazole, Viniconazole, Voriconazole, 1-(4-Chlorphenyl)-2-(1H-1,2,4-triazol-1-yl)cycloheptanol;
10) Inhibitoren der Zellwandsynthese: z.B. Benthiavalicarb, Dimethomorph, Flumorph, Iprovalicarb, Mandipropamid, N-[2-(4-{[3-(4-Chlorphenyl)prop-2-yn-l-yl]oxy}-3-methoxyphenyl)ethyl]-N-2-(methylsulfonyl)valinamide, Polyoxins, Polyoxorim, Validamycin A, Valiphenal;
11) Inhibitoren der Melanin-Biosynthese: z.B. Carpropamid, Diclocymet, Fenoxanil, Phthalide, Pyroquilon, Tricyclazole;
12) Resistenzinduktoren: z.B. Acibenzolar-S-methyl, Probenazole, Tiadinil;
13) Verbindungen mit Multisite-Aktivität: z.B. Bordeaux Mixture, Captafol, Captan, Chlorothalonil, Copper hydroxide, Copper naphthenate, Copper oxide, Copper oxychloride, Copper sulphate, Dichlofluanid, Dithianon, Dodine, Dodine free base, Ferbam, Fluorofolpet, Folpet, Guazatine, Guazatine acetate, Iminoctadine, Iminoctadine albesilate, Iminoctadine triacetate, Mancopper, Mancozeb, Maneb, Metiram, Metiram Zinc, Oxine-Copper, Propineb, Sulphur und Schwefelzubereitungen wie z.B. Calcium polysulphide, Thiram, Tolylfluanid, Zineb, Ziram;
14) eine Verbindung aus der folgenden Liste: 2,3,5,6-Tetrachlor-4-(methylsulfonyl)pyridine, 2,3-Dibutyl-6-chloro-thieno[2,3-d]pyrimidin-4(3H)one, 2-Butoxy-6-iod-3-propyl-benzo-pyran-4-on, 2-chloro-N-(2,3-dihydro-1,1,3-trimethyl-1H-inden-4-yl)-3-pyridincarboxamide, 3,4,5-Trichlorpyridine-2,6-dicarbonitrile, 3-[5-(4-Chlorphenyl)-2,3-dimethyl-isoxazolidin-3-yl]pyridine, 8-hydroxyquinoline-sulfate, Benthiazole, Bethoxazin, Capsimycin, Carvone, Chinomethionat, Cufraneb, Cyflufenamid, Cymoxanil, Cyprosulfamide, Dazomet, Debacarb, Dichlorophen, Diclomezine, Dicloran, Difenzoquat, Difenzoquat methylsulphate, Diphenylamine, Ferimzone, Flumetover, Fluopicolide, Fluoroimide, Flusulfamide, Fosetyl-Al, Fosetyl-calcium, Fosetyl-natrium, Hexachlorobenzene, Irumamycin, Isotianil, Methasulfocarb, Methyl isothiocyanate, Metrafenone, Mildiomycin, N-(4-Chlor-2-nitrophenyl)-N-ethyl-4-methylbenzene-sulfonamide, N-(4-Chlorbenzyl)-3-[3-methoxy-4-(prop-2-yn-1-yloxy)phenyl]propanamide, N-(6-methoxy-3-pyridinyl)-cyclopropanecarboxamide, N-[(4-Chlorphenyl)(cyano)methyl]-3-[3-methoxy-4-(prop-2-yn-1-yloxy)-phenyl]-propanamide, N-[(5-Brom-3-chlorpyridin-2-yl)methyl]-2,4-dichlornicotinamide, N-[1-(5-Brom-3-chlorpyridin-2-yl)ethyl]-2,4-dichlornicotinamide, N-[1-(5-Brom-3-chlorpyridin-2-yl)ethyl]-2-fluoro-4-iodonicotinamide, Natamycin, Nickel-dimethyldithiocarbamate, Nitrothal-isopropyl, Octhilinone, Oxyfenthiin, Pentachlorophenol and salts, Phosphorous acid and its salts, Propamocarb-Fosetyl, Propanosine-sodium, Proquinazid, Pyrrolnitrine, Quintozene, S-allyl-5-amino-2-isopropyl-4-(2-methylphenyl)-3-oxo-2,3-dihydro-1H-pyrazole-1-carbothioate, Tecloftalam, Tecnazene, Triazoxide, Trichlamide, Zarilamid zur Herstellung eines Mittels gegen Mykosen bei Fischen und Wirbellosen und all deren Entwicklungsstadien, hervorgerufen durch Pilze der Gattungen *Saprolegnia, Achlya, Aphanomyces.*

4. Verwendung von mindestens einem Fungizid ausgewählt aus folgender Gruppe Benthiavalicarb, Bixafen, Dimetomorph, Epoxiconazole, Ethaboxam, Fluopicolide, Fluopyram, Fosetyl-Al, Iprovalicarb, Isotianil, Mandipropamid, N-[2-(1,3-Dimethylbutyl)phenyl]-5-fluor-1,3-dimethyl-1H-pyrazol-4-carboxamid, 5-Chlor-6-(2,4,6-trifluorphenyl)-7-(4-methylpiperidin-1-yl)[1,2,4]triazolo[1,5-a]pyrimidin, Propamocarb, Propamocarb hydrochloride, Prothioconazole, Pyrimethanil, Tebuconazole und Zoxamide zur Herstellung eines Mittels gegen Mykosen bei Fischen und Wirbellosen und all deren Entwicklungsstadien, hervorgerufen durch Pilze der Gattungen *Saprolegnia, Achlya, Aphanomyces.*

5. Verfahren zur Prophylaxe und Therapie von Mykosen bei Fischen und Wirbellosen und deren Entwicklungsstadien, **dadurch gekennzeichnet, dass** befruchtete Eier, junge heranwachsende Fische und/oder erwachsene Tiere mit einem Mittel gemäß Anspruch 1 oder 2 durch Zudosierung zum Wasser oder Futter oder durch Direktapplikation behandelt werden.
